Europäisches Patentamt

**European Patent Office**

Office européen des brevets

(19)

(11) Publication number: **0 065 409**
A2

(12)

# EUROPEAN PATENT APPLICATION

(21) Application number: **82302404.7**

(22) Date of filing: **11.05.82**

(51) Int. Cl.³: **G 01 N 21/47, G 01 N 33/54**

(30) Priority: **12.05.81 JP 70178/81**

(43) Date of publication of application: **24.11.82**
Bulletin 82/47

(84) Designated Contracting States: **DE FR GB**

(71) Applicant: **OLYMPUS OPTICAL CO., LTD.,**
**43-2, 2-chome, Hatagaya Shibuya-ku, Tokyo (JP)**

(72) Inventor: **Kato, Masahiko, No. 37-3, Shimoyotsugi,**
**Akigawa City Tokyo (JP)**

(74) Representative: **Fane, Christopher Robin King et al,**
**HASELTINE LAKE & CO. Hazlitt House 28 Southampton**
**Buildings Chancery Lane, London, WC2A 1AT (GB)**

(54) **Apparatus for detecting a particle agglutination pattern.**

(57)    A laser light beam emitted from a laser light source is deflected by a swingable mirror by a given angle and a deflected laser light beam is focused onto bottom surfaces of the reaction vessels formed in a microplate in a matrix form by a first Fresnel's lens having an object focal point situating at an axis about which the mirror is swung and an image focal point situating at the bottom surfaces of the reaction vessels. By swinging the mirror, particle agglutination patterns formed on the bottom surfaces of the reaction vessels arranged in a row are successively scanned by the laser light spot. The light beam passing through the reaction vessel is focused onto a photoelectric converting device by means of a second Fresnel's lens to produce an electrical signal representing the particle agglutination patterns. A shielding plate having a plurality of openings formed therein is arranged in front of the second Fresnel's lens.

EP 0 065 409 A2

0065409

## APPARATUS FOR DETECTING
## A PARTICLE AGGLUTINATION PATTERN

This invention relates to an apparatus for detecting a particle agglutination pattern formed on a bottom surface of a reaction vessel in response to immunological agglutination reaction, and more particularly to an apparatus for identifying various kinds of blood types with the aid of agglutination patterns of blood corpuscles and for detecting various kinds of antibodies and antigens in sample solutions.

In a conventional method of identifying blood types, for example, which has heretofore been proposed, use was made of a winecup-shaped reaction vessel into which was quantitatively introduced a sample solution, i.e. 2 to 5% of test blood corpuscles suspended in saline solution, and a specified antiserum, i.e. A-type or B-type antiserum. Then, the mixture was remained stationary for reaction between blood corpuscles and antiserum. Subsequently, it was centrifuged to sediment the blood corpuscles. Then, the reaction vessel was rapidly wobbled such that the sedimented blood corpuscles were forcedly separated one from the other and then relatively slowly wobbled so as to collect the clumped compositions in the center portion of the base surface of the vessel and form settling patterns, thereby photometrically detecting these patterns.

Such conventional blood type identifying method in which sedimentation is effected and then the reaction vessel is rapidly wobbled so as to separate the sedimented blood corpuscles from the base surface of the vessel can only be applied to the analysis of regular ABO blood type which shows strong agglutination, but could not be applied to many other immunological agglutination reactions which show weak agglutination, for example, a method of determining Rh blood subtype or detecting various kinds of incomplete antibodies. That is, if the agglutination reaction is weak, the blood corpuscles or the like which have been clumped together become separated one from other when the reaction vessel is wobbled, and as a result, the particles are not collected in the center portion of the reaction vessel.

In order to detect and measure HBs antigen, a method has been proposed which makes use of a plastic plate, called as microplate, provided with a number of wells, i.e. reaction vessels each having a conical base surface. This conventional method makes use of a microplate having 10×12 wells, for example, and detects and prescribes the HBs antigen by the following procedure.

1). Buffer solution specially prescribed for R-PHA method is introduced into each well of the microplate one drop (0.025 mℓ) at a time.

2) A test serum (0.025 mℓ) is added to the first well of a row.  By using a diluter, the doubling dilution is performed along the row up to the last (tenth) well.

3) One drop of R-PHA buffer (0.025 m$\ell$) is added to a first row and one drop of R-PHA inhibition solution is added to a second row.

4) After the mixture thus treated have been sufficiently agitated by a micromixer for 10 seconds, incubation is effected for one hour at 37°C.

5) A drop of R-PHA cells of 1% suspension (0.025 m$\ell$) is added to each well.

6) The mixtures are agitated by the micromixer for ten seconds to suspend the R-PHA cells uniformly.

7) After the mixtures thus treated have been made stationary at room temperature for one hour, agglutination patterns are detected.

In this known method, since the reaction vessels, i.e. the wells in the microplate are not subjected to vibration before the measurement, the sedimented blood cells are not separated and thus relatively clear agglutination patterns are formed even if the agglutination reaction is rather weak.

In order to detect photoelectrically the particle pattern formed on the bottom surface of reaction vessel, use is generally made an incandescent lamp such as tungsten lamp.  The particle patterns formed in the microplate are illuminated by light emitted from the lamp by means of a diffusion plate and an image of the particle pattern is formed on a light detector having suitable light receiving regions or the light passing through the particle pattern is detected by the light detector. In most cases, the particle patterns formed on the bottom surfaces of reaction vessels have clearly differentiated between a uniformly deposited particle pattern in case of the agglutination reaction and a centrally collected particle pattern in case of non-agglutination reaction. Therefore, the particle patterns can be detected accurately with the aid of the known detecting apparatus. However, sometimes particle patterns are not formed clearly and intermediate patterns between the uniformly deposited particle pattern and the centrally collected particle pattern are often formed. The detecting precision of known detecting apparatus is not so high that such intermediate particle patterns can be precisely detected.

In a Japanese Patent Application Laid-open Publication No. 2,562/78 published on January 12, 1981, there has been proposed a detecting apparatus in which a particle pattern formed on a bottom surface of a reaction vessel is scanned by means of a light spot. That is to say, a scanning light beam is made incident upon the particle pattern and an image of an illuminated part of the pattern is projected by means of a lens onto a light detector. By means of such an apparatus it is possible to detect the particle pattern precisely. However, if such an apparatus is applied to a microplate having a number of reaction vessels formed therein in a matrix form, there must be provided a plurality of scanning light sources, lenses and light detectors, the number of which is equal to that of the reaction vessels formed in one row. That is to say, when use is made of a microplate in which ten reaction vessels are formed in each row, there must be arranged ten light sources, ten lenses and ten light detectors. Therefore, the construction of the optical system becomes very large and complicated. Further, ten signal processing channels must also be provided.

The present invention has for its object to provide an apparatus for detecting particle patterns on a plurality of reaction vessels formed in a microplate, which apparatus can detect the particle patterns in an accurate and precise manner.

An embodiment of the invention can provide a particle pattern detecting apparatus which is simple in construction and small in size and requires a small . number of optical elements.

According to the invention an apparatus for detecting photoelectrically particle agglutination patterns formed on bottom surfaces of reaction vessels arranged in a microplate in a matrix form, at least a part of each bottom surface being inclined with respect to the horizontal plane, comprises

means for emitting a light beam which is deflected in a plane including one row of reaction vessels in the microplate over a predetermined angle;

first optical means having an object focal point situating at said light beam emitting means and an image focal point situating at the bottom surfaces of the reaction vessels in the row to converge the light beam deflected over said predetermined angle within a length of the row;

second optical means having an object focal point situating at the bottom surfaces of the reaction vessels in the row and an image focal point; and

photoelectric converting means arranged at said image focal point of the second optical means for converting the light beam emanating from said second optical means into an electrical signal;

whereby the particle agglutination patterns formed on the bottom surfaces of the reaction vessels in the row are successively scanned by the light beam to obtain successively the electrical signal representing the particle agglutination patterns formed on the bottom surfaces of the reaction vessels in the row.

Embodiments of the invention will now be described in detail with reference to the accompanying drawings, wherein:

Fig. 1 is a schematic cross sectional view showing an embodiment of the particle agglutination pattern detecting apparatus according to the invention;

Fig. 2 is a schematic view illustrating a part of an optical system of the apparatus shown in Fig. 1;

Fig. 3 is a plan view depicting a microplate;

Fig. 4 is a plan view showing a shielding plate of the apparatus of Fig. 1;

Fig. 5 is an enlarged cross sectional view illustrating a reaction vessel and a particle agglutination pattern; and

Fig. 6 is a signal waveform obtained by scanning a bottom surface of the reaction vessel.

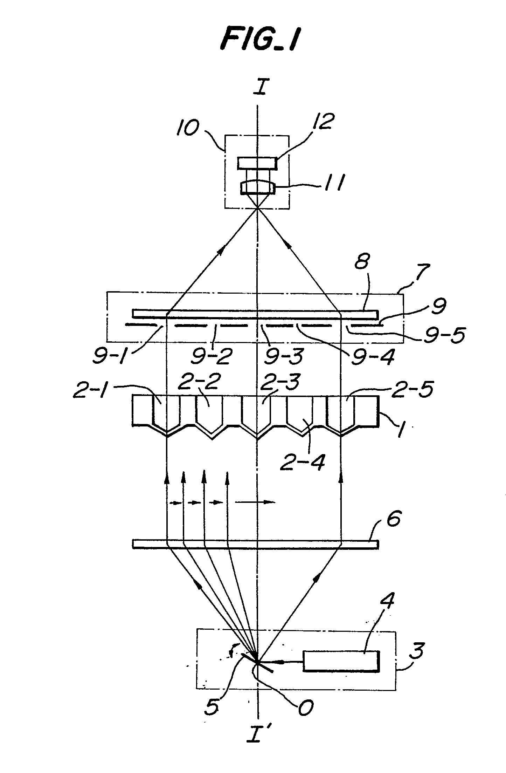

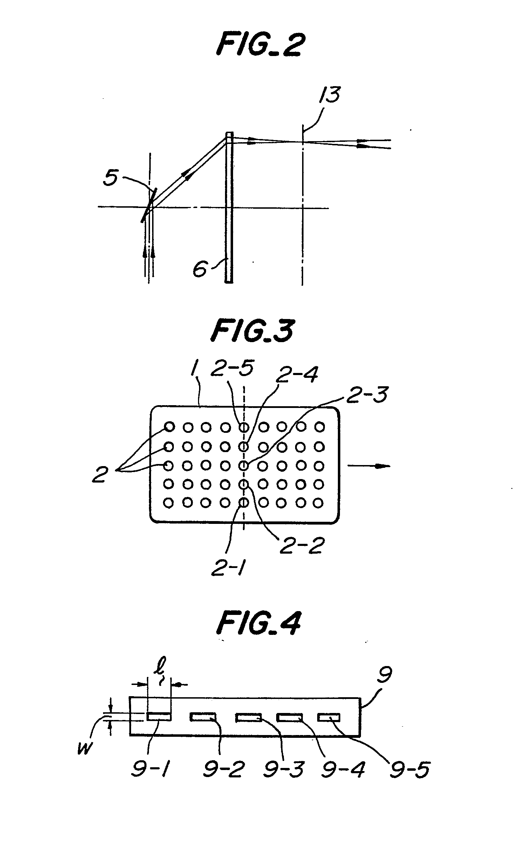

Fig. 1 is a schematic cross sectional view showing an embodiment of the apparatus for detecting particle agglutination patterns formed on bottom surfaces of reaction vessels arranged in a microplate in a matrix form. As shown in Fig. 3, a microplate 1 is made of transparent material and has a number of reaction vessels 2 formed therein in a matrix form. In this embodiment, the microplate 1 has 5×9 reaction vessels, i.e. five reaction vessels 2-1 to 2-5 in a row. According to the present embodiment the particle agglutination patterns formed on the reaction vessels 2-1 to 2-5 in the row are successively scanned by a light beam. For this purpose, the apparatus comprises light source means 3 including a laser light source 4 and a swingable mirror 5, first optical means 6 consisting of a first Fresnel's lens, second optical means 7 including a second Fresnel's lens 8 and a shielding plate 9 having apertures or openings 9-1 to 9-5 formed therein, and a photoelectric converting means 10 including a lens 11 and a light detector 12. The mirror 5 is journaled about an axis O and is swung by a predetermined angle. The first Fresnel's lens 6 has an object focal point situating at the axis O and an image focal point situating at the bottom surfaces of the reaction vessels 2-1 to 2-5. Therefore, a laser light beam emitted from the laser light source 4 and deflected by the mirror 5 is refracted by the first Fresnel's lens 6 into a direction parallel to an optical axis I-I'. Further, as illustrated in Fig. 2, the light beam is converged by the first Fresnel's lens 6 and is focused onto a plane 13 of the bottom surfaces of the reaction vessels 2-1 to 2-5. In this manner, the particle agglutination patterns formed on the conical bottom surfaces of reaction vessels 2-1 to 2-5 are successively scanned by a laser light spot. The deflection angle of the swingable mirror 5 is so determined that all of the reaction vessels 2-1 to 2-5 in the row can be scanned. The second Fresnel's lens 8 has an object focal point situating on the plane 13 of the bottom surfaces of reaction vessels 2-1 to 2-5 and an image focal point situating at an entrance of the photoelectrically converting means 10. In the present embodiment, the lens 11 has an object focal point situating at the image focal point of the second Fresnel's lens 8 and thus converging light emanating from the second Fresnel's lens 8 is converted into parallel light which is then made incident upon the light detector 12 perpendicularly thereto. As shown in Fig. 4, the shielding plate 9 has the openings 9-1 to 9-5 each of which has a length $\ell$ corresponding to a length or a diameter of the opening of the reaction vessel and has a width $w$ corresponding to a diameter of the light beam.

In the present embodiment, the laser light beam emitted from the laser light source 4 is reflected by the mirror 5 and is made incident upon the microplate 1 from its lower surface. As the mirror 5 is swung about the axis O the particle agglutination pattern formed on the bottom surface of the reaction vessel 2-1 is scanned by the very small light spot. In this manner, the particle agglutination pattern can be detected with a high resolution. The light beam emanating from the reaction vessel 2-1 is transmitted through the opening 9-1 of the shielding plate 9 and is converged by the second Fresnel's lens 8. Then the light beam is made incident upon the light detector 12 via the lens 11. Therefore, even if a sensitivity of the light detector 12 varies in dependent upon an incident angle, the detection can be effected without being influenced by the variation of the sensitivity. It should be noted that when the light detector has a larger variation in sensitivity due to incident positions than that due to incident angles, it is preferable to remove the lens 11. The shielding plate 9 has such a function that it can pass exclusively the laser beam emanating from the reaction vessel and cut any stray light emanating from any other portions and thus, an electrical signal supplied from the light detector 12 can have a high signal to noise ratio. The shielding plate 9 can further improve a raising characteristic of the electrical signal and a generation of a start pulse for processing the electrical signal in a succeeding circuit. In this manner, the particle agglutination patterns formed on the bottom surfaces of the reaction vessels 2-1 to 2-5 in the row can be successively scanned. After scanning the patterns in the row, the microplate 1 is moved in a direction perpendicular to the row by one pitch so as to bring reaction vessels in a next row into the detecting plane.

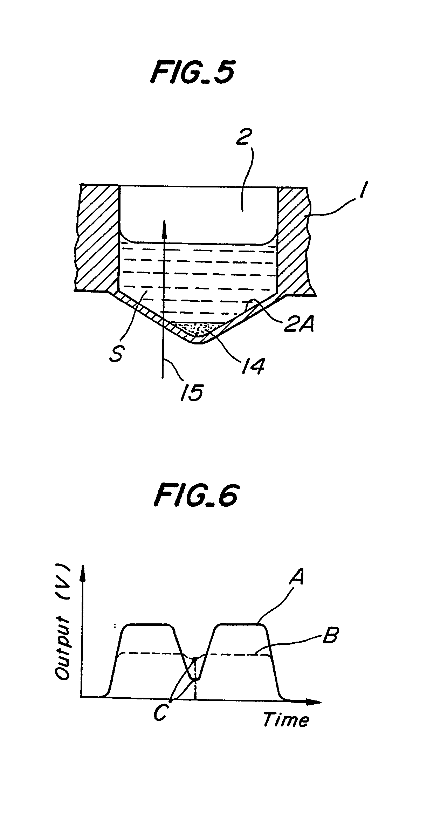

Fig. 5 is an enlarged cross sectional view of the reaction vessel 2 formed in the microplate 1 and Fig. 6 is a signal waveform obtained by scanning the reaction vessel 2. In Fig. 5 a test liquid S is contained in the vessel 1 and the centrally collected particle pattern 14 is formed on the bottom surface due to the absence of the agglutination reaction. Such a pattern may be produced when the test liquid S has been formed by mixing 50 $\mu\ell$ of a sample blood cell suspension of 2% and antiserum reagent of the same blood type as the sample

- 11 -

0065409

blood and the test liquid is remained stationary for several tens minutes. When the laser beam 15 scans the bottom surface 2A of the reaction vessel 2, it is absorbed by the blood cells and thus, the light detector 12 produces an output signal shown in Fig. 6 by a solid curve A. When the uniformly deposited pattern is formed due to the agglutination reaction, the light detector 12 produces an output signal illustrated in Fig. 6 by a broken curve B. It should be noted that points C on the curves A and B correspond to the lowermost center of the conical bottom surface 2A. Due to the shielding effect of the plate 9, the output of the light detector 12 becomes substantially zero. In embodiments of the invention it is possible to discriminate not only a uniformly collected pattern and a centrally deposited pattern, but also an intermediate pattern. In order to improve a signal to noise ratio of the output signal it is preferable to select a wavelength of the laser beam within an absorbing spectrum range of the blood cells, particularly to the peak absorbing wavelength. For instance, in case of using an Ar laser it is preferable to use a wavelength of 4,880 Å and for He-Cd laser, a wavelength of 4,416 Å may be advantageously used.

In the particle pattern detecting apparatus in embodiments of the invention, a plurality of the reaction vessels formed in the microplate can be successively scanned by the laser beam spot and thus, it is possible to detect the particle agglutination pattern in an accurate and precise manner.  Further, since the light source, the optical systems, the light detector and the signal processing channel can be used in common for all the reaction vessels, the apparatus can be made simple in construction and small in size, and the particle agglutination patterns can be detected in a short time.  ·

The present invention is not limited to the embodiments explained above, but many modifications may be conceived by those skilled in the art within the scope of the invention.  For instance, the first and second Fresnel's lenses may be replaced by hologram lens or ordinary lens. Further, the laser beam may be made incident upon the reaction vessel from above.  The openings formed in the shielding plate may be of any shape such as circle. Further, the light detector may be formed by a plurality of divided elements or an array of a number of photoconverting elements.  Moreover, the deflected light beam may be produced by any deflecting means other than the swingable mirror and the laser light source may be replaced by other light sources such as a tungsten lamp.

0065409

## Claims

1.    An apparatus for detecting photoelectrically particle agglutination patterns formed on bottom surfaces of reaction vessels arranged in a microplate in a matrix form, at least a part of each bottom surface being inclined with respect to the horizontal plane, comprising

means for emitting a light beam which is deflected in a plane including one row of reaction vessels in the microplate over a predetermined angle;

first optical means having an object focal point situating at said light beam emitting means and an image focal point situating at the bottom surfaces of the reaction vessels in the row to converge the light beam deflected over said predetermined angle within a length of the row;

second optical means having an object focal point situating at the bottom surfaces of the reaction vessels in the row and an image focal point; and

photoelectric converting means arranged at said image focal point of the second optical means for converting the light beam emanating from said second optical means into an electrical signal;

whereby the particle agglutination patterns formed on the bottom surfaces of the reaction vessels in the row are successively scanned by the light beam to obtain successively the electrical signal representing the particle agglutination patterns formed on the bottom surfaces of the reaction vessels in the row.

2.    An apparatus according to claim 1, wherein said light beam emitting means comprises a laser light source and a reflection mirror arranged swingably about an axis which extends perpendicularly to said plane and situates at the object focal point of the first optical means.

3.    An apparatus according to claim 1, wherein said first optical means comprises a Fresnel's lens.

4.    An apparatus according to claim 1, wherein said second optical means comprises a second Fresnel's lens.

5.    An apparatus according to claim 4, wherein said second optical means further comprises a shielding plate having a plurality of openings formed therein, each opening corresponding to each reaction vessel in the row.

6.    An apparatus according to claim 5, wherein said opening is formed in an elongated slit having a length corresponding to a diameter of the bottom surface and a width corresponding to a diameter of the light beam spot.

7.    An apparatus according to claim 1, wherein said photoelectric converting means comprises a lens having an object focal point situating at the image focal point of the second optical means and a light detector arranged to receive the light emanating from the lens.

0065409

8. An apparatus according to claim 1, wherein said light beam emitting means comprises a laser light source for generating a laser light beam having a wavelength within an absorbing spectrum range of particles forming the pattern.

9. An apparatus according to claim 8, wherein said laser light source is consisting of Ar laser emitting a laser light beam of a wavelength of 4,880 Å.

10. An apparatus according to claim 8, wherein said laser light source is consisting of He-Cd laser emitting a laser light beam of a wavelength of 4,416 Å.

11. An apparatus according to any one of claims 1 to 10, wherein said light beam emitting means and first optical means are arranged underneath the microplate, and said second optical means and photoelectric converting means are arranged above the microplate.

12. Apparatus for detecting particle agglutination patterns formed on bottom surfaces of transparent-based reaction vessels arranged in a row, comprising a light source for transmitting a narrow beam of light through the base of a vessel, and photoelectric detection means for receiving the light beam and detecting the intensity thereof, wherein the said light beam is moved relative to and in a plane containing the said row of reaction vessels when the device is in use, to scan particle agglutination patterns in successive reaction vessels and produce successive electrical outputs from the detection device corresponding to agglutination patterns in the respective vessels.

**FIG.1**

**FIG.2**

**FIG.3**

**FIG.4**

## FIG.5

## FIG.6